# EUROPEAN PATENT APPLICATION

(11) **EP 4 711 466 A1**
(43) Date of publication of application: **18.03.2026**
(21) Application number: 24796114.7
(22) Date of filing: 24.04.2024
(51) Int. Cl.: C12N 15/67, C12N 15/113, C12N 5/10, A61K 31/7088

(54) **ENGINEERED MRNA AND USE THEREOF**

(30) Priority: 28.04.2023 CN 202310484721
(71) Applicant: XIANWEI (HAINAN) BIOTECHNOLOGY CO. LTD., Haikou, Hainan 570311 (CN)
(72) Inventor: YU, Tianqi, Shanghai 201318 (CN); MA, Guanggang, Shanghai 201318 (CN); LIU, Amy, Shanghai 201318 (CN); TIAN, Jialun, Beijing 101400 (CN)
(74) Representative: KATZAROV S.A.
(86) International application number: PCT/CN2024/089552
(87) International publication number: WO 2024/222737

(57) **Abstract**

Provided are an engineered mRNA and a method for improving protein expression using same. Also provided is the use of such engineered mRNA in gene therapy and/or genetic vaccination.

## Description

### TECHNICAL FIELD

The present disclosure relates to the field of molecular biology, in particular to a series of engineered mRNAs and a method for improving protein expression using same.

### BACKGROUND

mRNA molecules all share common features: the 5' end contains a specially modified base called a cap, the 3' end has a segment of adenines called a polyA tail, as well as coding and non-coding regions. The coding region is translated into a protein, while the non-coding region (UTR) located at the 5' end is called 5'UTR, and that at the 3' end is called 3'UTR. There are a variety of regulatory elements inside the UTR. The 5'UTR is crucial for ribosome recruitment to the messenger RNA and start codon selection, playing a significant role in controlling protein translation efficiency. The 3'UTR regulates multiple aspects including nuclear export, cytoplasmic localization, mRNA stability, and the like.

*In vitro* transcribed (IVT) mRNA, as a technological foundation for vaccines or therapies, has gradually become a focus of attention. This artificially synthesized messenger ribonucleic acid offers great flexibility in both production and application and can transiently express proteins in a manner similar to natural mRNA. Any protein can be encoded and expressed by *in vitro* transcribed mRNA, enabling the development of preventive and therapeutic vaccines for diseases, such as infection and cancer, as well as protein replacement therapies. Since generating different proteins only requires changing the base sequence of RNA while its physicochemical properties remain largely unaffected, different products can be produced using the same production process without any adjustments to instruments or equipment, saving time and reducing costs compared with various other platforms.

IVT mRNA has several important distinctions from other nucleic acid or protein-based therapeutic approaches. It does not need to enter the cell nucleus to function; once it enters the cytoplasm, the mRNA is immediately translated into a protein. In contrast, DNA therapies require entry into the cell nucleus and need to be transcribed into RNA to become functional. Furthermore, unlike plasmid DNA and viral vectors, IVT mRNA-based therapies do not integrate into the genome, thus posing no risk of insertional mutations or recombination. For most pharmaceutical applications, IVT mRNA is only transiently active and is completely degraded via physiological metabolic pathways, which is also advantageous. In addition, mRNA vaccines, by binding to pattern recognition receptors, can be designed to be self-adjuvanting, a characteristic lacking in peptide and protein-based vaccines.

Due to its numerous technical advantages, IVT mRNA has become an emerging industry. However, the protein expression levels *in vivo* following IVT mRNA delivery are inconsistent. Therefore, improving mRNA expression efficiency is urgently needed. This efficiency is largely related to the 5'UTR, necessitating the screening of highly efficient 5'UTRs to meet the demand for high-efficiency mRNA expression.

### SUMMARY

The present disclosure provides an engineered mRNA to meet the demand for high-efficiency expression. The present disclosure also provides a nucleic acid vector encoding the mRNA, a lipid nanoparticle comprising the mRNA, a pharmaceutical composition, and related use thereof for treating or preventing a disorder.

In a first aspect, the present disclosure provides an engineered mRNA, and the engineered mRNA comprises:
a first nucleic acid sequence, wherein the first nucleic acid sequence comprises at least one engineered 5' untranslated region (5'UTR) sequence;
a second nucleic acid sequence, wherein the second nucleic acid sequence comprises at least one open reading frame (ORF); and
a third nucleic acid sequence, wherein the third nucleic acid sequence comprises at least one 3' untranslated region (3'UTR) sequence.

In some embodiments, the engineered 5'UTR sequence comprises a sequence represented by formula (I) or formula (II) below: 5'-GG(N)ₙGCCACC-3' (I) (SEQ ID NO: 23); 5'-GG(H)ₙGCCACC-3' (II) (SEQ ID NO: 24), wherein
each N is independently selected from A, U, G, or C;
each H is independently selected from A, U, or C;
n represents the number of N or H and is any integer of 30-70;
preferably, formula (I) or formula (II) has a length of 60 nt or 70 nt, a GC content of 50-60%, and a minimum free energy of 0.

Preferably, n is 41, 52, or 62; preferably, when n is 52 or 62, in the direction from 5' to 3', the 1st N is selected from A or G, the 2nd N is selected from A or U, and the 4th, 5th, 15th, 27th, 35th, 41st, 46th, 47th, and 48th N are selected from A or C.

In some embodiments, the engineered 5'UTR sequence is selected from the group comprising the following: SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, or a sequence having at least 90% identity to the sequences set forth in SEQ ID NOs: 1-10 or having 1, 2, 3, or more nucleotide insertions, deletions, and/or substitutions.

Preferably, the 5'UTR sequence is suitable for increasing protein production from the mRNA.

In some embodiments, the 3'UTR sequence is selected from SEQ ID NO: 11.

Preferably, the at least one 3'UTR sequence and the at least one 5'UTR sequence act synergistically to increase protein production from the mRNA.

In some embodiments, the 5'UTR sequence, the open reading frame, and the 3'UTR sequence are heterologous.

In some embodiments, the open reading frame encodes a target protein, the target protein is a protein with biological activity or a protein with chromogenic/indicator functions, and the source of the target protein includes, but is not limited to, viruses, bacteria, and eukaryotes.

In some embodiments, the mRNA further comprises a 5' cap structure and a polyA sequence.

In a second aspect, the present disclosure provides a vector, and the vector comprises a nucleic acid encoding the engineered mRNA.

In some embodiments, the vector is an RNA vector or a DNA vector.

In some embodiments, the vector is a plasmid vector or a viral vector.

Preferably, the vector may further comprise an expression control element.

In a third aspect, the present disclosure provides a cell, and the cell comprises the vector described above; preferably, the cell is a prokaryotic cell or a eukaryotic cell, including but not limited to a bacterium (*E. coli*), a fungus (yeast), an insect cell, or a mammalian cell.

In a fourth aspect, the present disclosure provides a composition, which comprises the engineered mRNA according to any one of the above and an mRNA delivery system; preferably, the delivery system comprises a lipid nanoparticle, a complex and a polymer nanoparticle, an exosome, a biomicrovesicle, protamine, and the like.

In a fifth aspect, the present disclosure provides a pharmaceutical composition, which comprises the engineered mRNA, the vector, the cell, or the composition described above, and a pharmaceutically acceptable carrier.

In a sixth aspect, the present disclosure provides use of the engineered mRNA, the vector, the cell, the composition, or the pharmaceutical composition described above in preparing a medicament for treating or preventing a disease by administering to a subject in need thereof.

In a seventh aspect, the present disclosure provides a method for treating or preventing a disorder, and the method comprises administering to a subject in need thereof the engineered mRNA, the vector, the cell, the composition, or the pharmaceutical composition described above.

In an eighth aspect, the present disclosure provides a method for treating or preventing a disorder, and the method comprises transfecting a cell with the engineered mRNA or the vector described above.

In a ninth aspect, the present disclosure provides the engineered mRNA, the vector, the cell, the composition, or the pharmaceutical composition described above for use as a medicament, a vaccine, or a gene therapy.

In a tenth aspect, the present disclosure provides a method for increasing protein expression, which comprises providing the engineered mRNA, the vector, or the cell described above to a cell or tissue.

### Terminology and Definitions

Unless otherwise defined in the present disclosure, scientific and technical terms in correlation with the present disclosure shall have the meanings as understood by those of ordinary skill in the art.

Furthermore, unless otherwise stated herein, terms used in the singular form herein shall include the plural form, and vice versa. More specifically, as used in this specification and the appended claims, unless otherwise clearly indicated, the singular forms "a", "an", and "the" include referents in the plural form.

In the text above and below, unless otherwise specified, the uppercase letters C, G, U, T, and A represent the base composition of a nucleotide.

The terms "include", "comprise", and "have" herein are used interchangeably and are intended to indicate the inclusion of a solution, implying that there may be elements other than those listed in the solution. Meanwhile, it should be understood that the descriptions "include", "comprise", and "have" used herein also provide the solution of "consist of". Illustratively, "a composition, comprising A and B" should be understood as the following technical solution: a composition consisting of A and B, and a composition containing other components in addition to A and B, all fall within the scope of the aforementioned "a composition".

The term "and/or" as used herein includes the meanings of "and", "or", and "all or any other combinations of elements linked by the term".

The term "nucleic acid" as used herein refers to a polymer composed of nucleotides (e.g., deoxyribonucleotides or ribonucleotides). "Nucleic acid" is used in its broadest sense and includes any compound and/or substance containing a polymer of nucleotides or a derivative or analog thereof. These polymers are often referred to as "polynucleotides". Thus, as used herein, the terms "nucleic acid" and "polynucleotide" are equivalent and may be used interchangeably. Exemplary nucleic acids or polynucleotides of the present disclosure include, but are not limited to, ribonucleic acids (RNAs), deoxyribonucleic acids (DNAs), DNA-RNA hybrids, RNAi inducers, RNAi agents, siRNAs, shRNAs, mRNAs, modified mRNAs, miRNAs, antisense RNAs, ribozymes, catalytic DNAs, RNAs that induce triple helix formation, threose nucleic acids (TNAs), glycol nucleic acids (GNAs), peptide nucleic acids (PNAs), locked nucleic acids (LNAs, including LNA having a β-D-ribose configuration, α-LNA having an α-L-ribose configuration (diastereomer of LNA), 2'-amino-LNA having 2'-amino functionalization, and 2'-amino-α-LNA having 2'-amino functionalization), or hybrids thereof.

The term "nucleoside" as used herein refers to a compound containing a sugar molecule (e.g., ribose in RNA or deoxyribose in DNA) or a derivative or analog thereof covalently linked to a nucleobase (e.g., purine or pyrimidine) or a derivative or analog thereof (also referred to herein as a "nucleobase"), but lacking an internucleoside linking group (e.g., a phosphate group).

The term "nucleotide" as used herein refers to a nucleoside covalently bonded to an internucleoside linking group (e.g., a phosphate group) or any derivative, analog, or modification thereof, which confers improved chemical and/or functional properties (e.g., binding affinity, nuclease resistance, chemical stability) to the nucleic acid or portion or fragment thereof.

The terms "ribonucleic acid" and "RNA" as used herein refer to a polymer composed of ribonucleotides. It is a nucleic acid molecule, i.e., a polymer composed of nucleotides. These nucleotides are usually adenosine-monophosphate, uridine-monophosphate, guanosine-monophosphate, and cytidine-monophosphate monomers linked to each other along the so-called backbone. The backbone is formed by a phosphodiester bond between the sugar, i.e., ribose, of the first adjacent monomer and the phosphate moiety of the second adjacent monomer. The specific succession of monomers is referred to as the RNA sequence. RNA can be generally obtained, for example, by transcription of a DNA sequence within a cell. In eukaryotic cells, transcription is typically carried out in the cell nucleus or mitochondria. Transcription of DNA often results in the production of so-called pre-mature RNA, which must be processed into so-called messenger RNA, often abbreviated to mRNA. For example, in eukaryotic organisms, the processing of pre-mature RNA includes various post-transcriptional modifications such as splicing, 5' capping, polyadenylation, export from the cell nucleus or mitochondria, and the like. The sum of these processes is also known as the maturation of RNA. Mature messenger RNA generally provides a nucleotide sequence that can be translated into a specific peptide or protein amino acid sequence. Typically, mature mRNA comprises a 5' cap, a 5'UTR, an open reading frame, a 3'UTR, and a polyA sequence. In addition to messenger RNA, there are some types of non-coding RNAs, which may be involved in regulating transcription and/or translation.

The term "mRNA" as used herein refers to messenger ribonucleic acid. mRNA may be naturally or non-naturally occurring or synthetic. For example, mRNA may comprise modified and/or non-naturally occurring constituents such as one or more nucleobases, nucleosides, nucleotides, or linkers. mRNA may comprise a cap structure, a 5' transcriptional leader, a 5' untranslated region, a start codon, an open reading frame, a stop codon, a chain-terminating nucleoside, a stem-loop, a hairpin, polyA, a polyadenylation signal, and/or one or more cis-regulatory elements. mRNA may have a nucleotide sequence encoding a polypeptide. Translation of mRNA, e.g., *in vivo* translation of mRNA in a mammalian cell, can produce a polypeptide. Traditionally, the basic components of natural mRNA molecules include at least one coding region, a 5'-untranslated region (5'UTR), a 3'UTR, a 5' cap, and a polyA sequence.

The term "untranslated region" or "UTR" as used herein relates to the mRNA portions upstream of the start codon and downstream of the stop codon, which are not translated and thus are referred to as the 5' untranslated region (5'UTR) and 3' untranslated region (3'UTR), respectively. These regions are transcribed with coding regions and are therefore exons when present in the mature mRNA.

The term "5' untranslated region" or "5'UTR" as used herein generally refers to a portion of mRNA that is located 5' (i.e., "upstream") of the open reading frame and that is not translated into a protein. 5'UTR is generally understood as a specific segment of messenger RNA (mRNA), and the messenger RNA is located at the 5' end of the open reading frame of the mRNA. Generally, the 5'UTR begins at the transcription start site and ends at one nucleotide before the start codon of the open reading frame. Preferably, the 5'UTR has a length of more than 20, 30, 40, or 50 nucleotides. The 5'UTR may comprise an element for controlling gene expression, which is also referred to as a regulatory element. The regulatory element may be, for example, a ribosome binding site. The 5'UTR may be post-transcriptionally modified, for example, by the addition of a 5'-cap modification. The 5'UTR of mRNA is not translated into an amino acid sequence. The 5'UTR sequence is generally encoded by the gene that is transcribed into the respective mRNA during gene expression. The genomic sequence is first transcribed into pre-mature mRNA, which contains optional introns. The pre-mature mRNA is then further processed into mature mRNA during maturation. This maturation process includes the following steps: 5' capping, splicing the pre-mature mRNA to remove optional introns, and 3' end modifications (such as polyadenylation of the 3' end of the pre-mature mRNA and optional endonuclease and/or exonuclease cleavage). Within the scope of the present disclosure, the 5'UTR corresponds to the mature mRNA sequence located between the start codon and, for example, the 5'-cap. Preferably, the 5'UTR corresponds to the sequence extending from a nucleotide located on the 3' side of the 5' cap (more preferably from a nucleotide immediately adjacent to the 3' side of the 5' cap) to a nucleotide located on the 5' side of the start codon of the protein-coding region (preferably to a nucleotide immediately adjacent to the 5' side of the start codon of the protein-coding region). The nucleotide immediately adjacent to the 3' side of the mature mRNA 5' cap typically corresponds to the transcription start site. The term "corresponds to" means that the 5'UTR sequence may be an RNA sequence in an mRNA sequence used to define the 5'UTR sequence, or a DNA sequence corresponding to the RNA sequence.

The term "3' untranslated region" or "3'UTR" as used herein generally refers to a portion of mRNA that is located 3' (i.e., "downstream") of the open reading frame and that is not translated into a protein. Generally, the 3'UTR is a portion of mRNA between the protein-coding region of the mRNA (open reading frame (ORF) or coding sequence (CDS)) and the polyA sequence. In the case of the present disclosure, the term 3'UTR may also comprise elements that are not encoded in the template from which the RNA is transcribed but are added post-transcriptionally during maturation, such as polyA sequences. The 3'UTR of mRNA is not translated into an amino acid sequence. The 3'UTR sequence is generally encoded by the gene that is transcribed into the respective mRNA during gene expression. The genomic sequence is first transcribed into pre-mature mRNA, which contains optional introns. The pre-mature mRNA is then further processed into mature mRNA during maturation. This maturation process includes the following steps: 5' capping, splicing the pre-mature mRNA to remove optional introns, and 3' end modifications (such as polyadenylation of the 3' end of the pre-mature mRNA and optional endonuclease and/or exonuclease cleavage). Within the scope of the present disclosure, the 3'UTR corresponds to the sequence located between the stop codon of the protein-coding region (preferably a position immediately following the 3' end of the stop codon) and the polyA sequence of the mRNA. The term "corresponds to" means that the 3'-UTR sequence may be an RNA sequence in an mRNA sequence used to define the 3'-UTR sequence, or a DNA sequence corresponding to the RNA sequence. Preferably, the 3'UTR has a length of more than 20, 30, 40, or 50 nucleotides.

The term "start codon" as used herein refers to the first codon of an open reading frame translated by a ribosome and consisting of a linked adenine-uracil-guanine nucleobase triplet. The start codon is described by the first letter codes of adenine (A), uracil (U), and guanine (G), and is usually simply written as "AUG". During translation initiation, the sequence comprising the start codon is recognized through complementary base pairing with the anticodon of the start tRNA (Met-tRNAᵢ^{Met}) bound to the ribosome. The open reading frame may comprise more than one AUG start codon.

The term "open reading frame (ORF)" as used herein refers to a fragment or region of an mRNA molecule that encodes a polypeptide. The ORF consists of a stretch of consecutive, nonoverlapping, and in-frame codons, beginning with a start codon and ending with a stop codon, and is translated by the ribosome.

The term "delivery system" as used herein refers to a carrier that delivers an mRNA molecule into a cell, including lipid nanoparticles, complex and polymer nanoparticles, exosomes, biomicrovesicles, protamine, and the like.

The term "base composition" as used herein refers to the proportion of total bases of a nucleic acid consisting of guanine + cytosine or thymine (or uracil) + adenine nucleotides.

As used herein, "expression" of a nucleic acid sequence refers to one or more of the following events: (1) production of an RNA template from a DNA sequence (e.g., by transcription); (2) processing of an RNA transcript (e.g., by splicing, editing, 5' cap formation, and/or 3' end processing); (3) translation of RNA into a polypeptide or protein; and (4) post-translational modification of a polypeptide or protein.

The term "heterologous" as used herein refers to a polynucleotide, gene, or nucleic acid molecule that is not derived from a host species. For example, as used herein, a "heterologous nucleic acid sequence" refers to a gene or nucleic acid sequence originating from a species different from that of the host organism into which the gene or nucleic acid sequence is introduced. When referring to a gene regulatory sequence, an auxiliary nucleic acid sequence used to manipulate the expression of a gene sequence (e.g., 5' untranslated region, 3' untranslated region, poly A addition sequence, etc.), or a nucleic acid sequence encoding a protein domain or protein localization sequence, "heterologous" means that the regulatory sequence, the auxiliary sequence, or the sequence encoding a protein domain or localization sequence originates from a source different from that of the gene. The regulatory nucleic acid sequence, the auxiliary nucleic acid sequence, or the nucleic acid sequence encoding a protein domain or localization sequence is inserted into the genome together with the gene.

The term "increase" as used herein may refer to any change that results in a higher level of gene expression, protein expression, symptom amount, disease, composition, condition, or activity. A substance is also understood to increase the level of a gene, protein, composition, or condition amount when the level of the gene, protein, composition, or condition amount is greater/higher than the output of the level of the gene, protein, composition, or condition amount in the absence of the substance. Further, for example, an increase may be a change in the symptoms of a disorder such that the symptoms are less than previously observed. An increase may be any individual increase, median increase, or average increase in a statistically significant amount of a condition, symptom, activity, or composition. Thus, an increase may be 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, or 100%, provided that the increase is statistically significant.

The term "identity" as used herein can be calculated as follows: to determine the percent "identity" between two amino acid sequences or two nucleic acid sequences, the sequences are aligned for optimal comparison purposes (e.g., gaps can be introduced into one or both of the first and second amino acid sequences or nucleic acid sequences for optimal alignment, or non-homologous sequences can be discarded for comparison). Amino acid residues or nucleotides at corresponding amino acid positions or nucleotide positions are then compared. When a position in the first sequence is occupied by the same amino acid residue or nucleotide at the corresponding position in the second sequence, the molecules are identical at this position.

The percent identity between two sequences varies with the identical positions shared by the sequences, taking into account the number of gaps that need to be introduced and the length of each gap for optimal alignment of the two sequences.

A mathematical algorithm can be used to compare two sequences and calculate the percent identity between the sequences. For example, the percent identity between two amino acid sequences is determined with the Needleman and Wunsch ((1970) J. Mol. Biol., 48 (3): 444-453) algorithm that has been integrated into the GAP program of the GCG software package, using the Blosum 62 matrix or PAM250 matrix and gap weight of 16, 14, 12, 10, 8, 6, or 4 and length weight of 1, 2, 3, 4, 5, or 6. For another example, the percent identity between two nucleotide sequences is determined with the GAP program of the GCG software package (available at www.gcg.com), using the NWSgapdna.CMP matrix and gap weight of 40, 50, 60, 70, or 80 and length weight of 1, 2, 3, 4, 5, or 6. A particularly preferred parameter set (and one that should be used unless otherwise stated) is a Blosum62 scoring matrix with a gap penalty of 12, a gap extension penalty of 4, and a frameshift gap penalty of 5.

The percent identity between two amino acid sequences or nucleotide sequences can also be determined with a PAM120 weighted remainder table, a gap length penalty of 12, and a gap penalty of 4, using the E. Meyers and W. Miller algorithm ((1989) CABIOS, 4: 11-17) that has been incorporated into the ALIGN program (version 2.0).

Additionally or alternatively, the nucleic acid sequences and protein sequences described in the present disclosure can be further used as "query sequences" to perform searches against public databases to, e.g., identify other family member sequences or related sequences. For example, such searches can be performed using the NBLAST and XBLAST programs (version 2.0) of Altschul et al., (1990) J. Mol. Biol., 215: 403-10. BLAST nucleotide searches can be performed using the NBLAST program, with a score of 100 and a word length of 12, to obtain nucleotide sequences homologous to the nucleic acid (SEQ ID NO: 1) molecule of the present disclosure. BLAST protein searches can be performed using the XBLAST program, with a score of 50 and a word length of 3, to obtain amino acid sequences homologous to the protein molecule of the present disclosure. To obtain gapped alignment results for the purpose of comparison, gapped BLAST can be used as described in Altschul et al., (1997) Nucleic Acids Res. 25: 3389-3402. When using the BLAST and gapped BLAST programs, the default parameters of the respective programs (e.g., XBLAST and NBLAST) can be used. See www.ncbi.nlm.nih.gov.

The term "vector" as used herein refers to a nucleic acid molecule capable of amplifying another nucleic acid to which it has been linked. The term includes vectors that serve as self-replicating nucleic acid structures, as well as vectors integrated into the genome of a host cell into which they have been introduced. Certain vectors are capable of directing the expression of nucleic acids to which they are operably linked.

The term "cell" as used herein refers to a cell into which an exogenous nucleic acid has been introduced, including the progeny of such a cell. Host cells include "transformants" and "transformed cells", which include primary transformed cells and progenies derived therefrom, regardless of the number of passages. Progenies may not be exactly the same as parent cells in terms of nucleic acid content and may contain mutations. Mutant progenies having the same function or biological activity that are screened or selected from the primary transformed cells are included herein.

The term "pharmaceutical composition" as used herein refers to a formulation that exists in a form allowing the biological activity of the active ingredient contained therein to be effective and does not contain additional ingredients having unacceptable toxicity to a subject to which the pharmaceutical composition is administered.

The term "subject" as used herein refers to an organism that receives treatment for a particular disease or disorder described in the present disclosure. Examples of subjects and patients include mammals, such as humans, primates (e.g., monkeys), or non-primate mammals, that receive treatment for a disease or disorder.

The term "treatment" as used herein refers to surgical or therapeutic treatment for the purpose of preventing or slowing (reducing) the progression of an undesired physiological or pathological change, e.g., a cancer, in a subject being treated. Beneficial or desired clinical results include, but are not limited to, alleviation of symptoms, decrease of severity of disease, stabilization (i.e., not worsening) of state of disease, delay or slowing of disease progression, amelioration or palliation of state of disease, and remission of state of disease (whether partial remission or complete remission), whether detectable or undetectable. Subjects in need of treatment include those already with a disorder or disease, as well as those who are susceptible to a disorder or disease, or those who intend to prevent a disorder or disease. When referring to terms such as slowing, alleviation, decrease, palliation, and remission, their meanings also include elimination, disappearance, nonoccurrence, etc.

The term "preventing" as used herein refers to reducing the likelihood of developing a disease, disorder, condition, or associated symptom (e.g., an infectious disease, such as a viral infection or a neoplastic disease, e.g., cancer).

The term "vaccine" as used herein refers to a formulation containing the engineered mRNA of the present disclosure, which is in a form that can be administered to a subject and induces a protective immune response sufficient to induce immunity to prevent and/or ameliorate infection and/or alleviate at least one symptom of the infection and/or enhance the efficacy of another dose of the vaccine. Generally, the vaccine comprises a conventional saline or buffered aqueous medium in which the composition of the present disclosure is suspended or dissolved. In this form, the composition of the present disclosure may conveniently be used to prevent, ameliorate, or otherwise treat infection. Upon introduction into a host, the vaccine is capable of eliciting an immune response that includes, but is not limited to, the production of antibodies and/or cytokines and/or the activation of CD8+ T cells, antigen-presenting cells, CD4+ T cells, dendritic cells, and/or other cellular responses.

### BRIEF DESCRIPTION OF THE DRAWINGS

FIG. 1 shows the method and parameters for artificial UTR screening;
FIG. 2 shows the pattern diagram of the T7-5'UTR-GFP vector, where T7 indicates the T7 promoter; 5'UTR indicates different 5'UTR nucleic acid sequences; 3'UTR indicates the 3-terminal UTR nucleic acid sequence; HDV ribozyme indicates the hepatitis delta virus ribozyme nucleic acid sequence;
FIG. 3 shows the plasmid map of pLVX-flag-NLS-T7 polymerase;
FIG. 4 shows the fluorescence images of GFP expressed by the top 10 different 5'UTRs at 44 h after co-transfection of different T7-5'UTR-GFP plasmids with the pLVX-flag-NLS-T7 polymerase plasmid;
FIG. 5 shows the results of the ranking of the top 10 different 5'UTRs based on mean fluorescence intensity (from highest to lowest) at 44 h after co-transfection of different T7-5'UTR-GFP plasmids with the pLVX-flag-NLS-T7 polymerase plasmid;
FIG. 6 shows the detection of the fluorescent expression of the target protein by different 5'UTR-mRNA molecules in 293T cells at 24 h;
FIG. 7 shows the detection of the mean fluorescence intensity of the target protein expressed by different 5'UTR-mRNA molecules in 293T cells;
FIG. 8 shows the detection of the expression of the target protein by different 5'UTR-mRNA molecules in BHK21 cells at 24 h;
FIG. 9 shows the detection of the mean fluorescence intensity of the target protein expressed by different 5'UTR-mRNA molecules in BHK21 cells;
FIG. 10 shows the positive rates of 293T cells with the expression of the target protein GFP by different 5'UTR-mRNAs at different time points as detected by flow cytometry, wherein FIG. 10A shows the expression rates of different 5'UTR-mRNAs in 293T cells at 24 h as detected by FACS;
FIG. 10B shows the expression rates of different 5'UTR-mRNAs in 293T cells at 48 h as detected by FACS; FIG. 10C shows the expression rates of different 5'UTR-mRNAs in 293T cells at 72 h as detected by FACS;
FIG. 11 shows the detection of the mean fluorescence intensity of the target protein GFP expressed by different 5'UTR-mRNA molecules in 293T cells at different time points;
FIG. 12 shows the expression of the target protein by different trans-self-replicating 5'UTR-NSP molecules in 293T cells at 24 h as detected by a fluorescence microscope;
FIG. 13 shows the expression of the target protein by different trans-self-replicating 5'UTR-NSP molecules in 293T cells at 24 h as detected by flow cytometry;
FIG. 14 shows the pattern diagrams of different 5'UTR-MICA/B molecular constructs;
FIG. 15 shows the mean fluorescence intensity of the target protein expressed by different 5'UTR-MICA/B molecules in BHK21 cells at 24 h as detected by flow cytometry.

### DETAILED DESCRIPTION

The present disclosure will be further described with reference to specific examples, and the advantages and features of the present disclosure will become more apparent with the description.

Experimental procedures without specified conditions in the examples are conducted according to conventional conditions or conditions recommended by the manufacturers. Reagents or instruments without specified manufacturers used herein are conventional products that are commercially available.

The examples of the present disclosure are exemplary only and do not limit the scope of the present disclosure in any way. It should be understood by those skilled in the art that changes or substitutions in details and forms may be made to the technical solutions of the present disclosure without departing from the spirit and scope of the present disclosure, and that these changes and substitutions shall fall within the protection scope of the present disclosure.

### Example 1. Screening of Artificial and Natural 5'UTRs

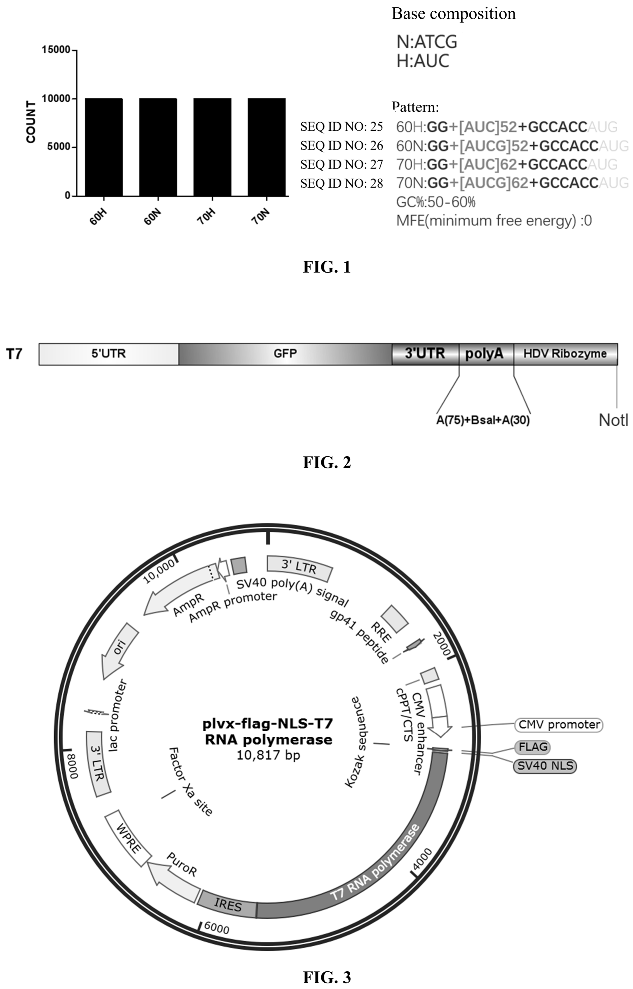

Screening of artificial 5'UTRs: 40000 (4 groups, with 10000 in each group) RNA molecules were generated by software, wherein as shown in FIG. 1, the base compositions were N (selected from A, U, C, or G) or H (selected from A, U, or C), the length was selected to be 60 nt or 70 nt, the minimum free energy (MFE) was set at 0, and the GC content was 50-60%. The obtained 40000 sequences were analyzed, and sequences containing uAUG were filtered out; software was used to analyze miRNA binding sites within the sequences. Sorting was performed according to the number of bound miRNAs from fewest to most, and finally 31 artificial sequences (5art1-5art31) were selected from them for subsequent experiments.

Screening of natural 5'UTRs: Human tissue protein expression chip data were downloaded from the HPA database, genes highly expressed in skeletal muscle, lymph nodes, and spleen were extracted, sequences with lengths of 20-100 nt, no internal ATG, and a minimum free energy greater than -15 kCal/Mol were screened, and finally 20 natural sequences (5nat1-5nat20) were selected for subsequent experiments.

### Example 2. Preliminary Screening of 5'UTRs

A GFP fluorescent protein expression detection method was used to preliminarily screen the 51 5'UTR sequences obtained in Example 1, and 5'UTR sequences with relatively good effects were selected. The specific method was as follows.

With fixed 3'UTR sequence (see SEQ ID NO: 11, the 3'UTR is a combination of two sequence elements derived from split amino-terminal enhancer (AES) mRNA and mitochondrially encoded 12S ribosomal RNA), PolyA sequence (SEQ ID NO: 12, from WHO International Nonproprietary Names Programme #11889), and HDV sequence (SEQ ID NO: 13, derived from hepatitis delta virus, NCBI accession number MG711762.1), T7-5'UTR-GFP sequences (see SEQ ID NO: 14 for the GFP sequence, derived from pVSV1(+)-GFP plasmid, NCBI sequence number MN153298.1) for the 51 5'UTRs described above were constructed, and the map is shown in FIG. 2. Meanwhile, pLVX-flag-NLS-T7 polymerase, a plasmid expressing T7 RNA polymerase, was also constructed, and the map is shown in FIG. 3.

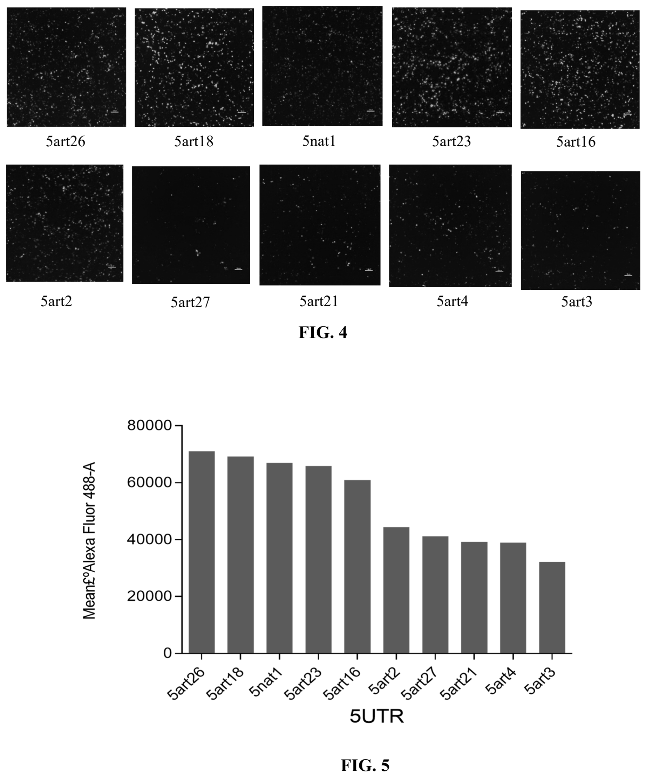

The pLVX-flag-NLS-T7 polymerase plasmid was co-transfected into 293T cells with the 51 T7-5'UTR-GFP plasmids, respectively. The transfection method was as follows: when cells in a 24-well plate grew to a density of ≥80%, transfection was performed using FuGENE^{®} HD transfection reagent (Promega, Cat. No.: E2311), and the FuGENE^{®} HD transfection reagent was brought back to room temperature before use. 50 µL of Opti-MEMI medium (Gibco, Cat. No.: 31985-062) was pipetted into a sterile centrifuge tube, 0.25 µg of the T7-5'UTR-GFP plasmid was added, and the mixture was well mixed. Subsequently, 0.25 µg of the pLVX-flag-NLS-T7 polymerase plasmid was added, and the mixture was well mixed. 1.5 µL of FuGENE^{®} HD transfection reagent was added to the diluted plasmid mixture, and the mixture was incubated at room temperature for 10 min before being added to the 24-well plate. Microscopic observation and flow cytometry detection for fluorescence intensity were performed at 44 h. The results for the top 10 in fluorescence intensity ranking are shown in FIG. 4 and FIG. 5. The RNA sequences of the 5'UTRs ranked top 10 in fluorescence intensity are shown in Table 1.

**Table 1. RNA sequences of 5'UTRs ranked top 10 in fluorescence intensity and sequences of each element of T7-5'UTR-GFP**

| SEQ ID NO. | Name | Sequence |
|---|---|---|
| 1 | 5art2 | GGAUAAAAGACUUCAGACACCACAAACGAAUUAGCCACGAUAACCCCCCCCG ACGCCACC |
| 2 | 5art3 | GGGACCCGACACGCAUCACAACACCUAUCAAAGCAAACCGACAACGCACCAC ACGCCACC |
| 3 | 5art4 | GGGAAAACUUUACACGACACGAGAGCAUCAGACGACAGGACCAUAAACACA ACCGCCACC |
| 4 | 5art16 | GGGAACACAGACAAGAACUCACGAACACCUAGCACCAUACACCAACGACAAU ACGCCACC |
| 5 | 5art18 | GGGAAAAAUACACACGAUCACACCUAAGCAGAAAAACCAAUCCAACCAACC GACCACAACGCACGCCACC |
| 6 | 5art21 | GGAUACACGCAUACCACGAGAGCUUACGACAACAACAACAUUAAUUGACCC GACGCCACC |
| 7 | 5art23 | GGAAGAACGACAAUUGACGCACCAUAGACGACCGAAAUAAUCCCGACCACA UUUGCCACC |
| 8 | 5art26 | GGGACAACGAGCAGCACACGAAGAUACACAACAGAAAGGAGAAAUCACACU AGCGCCACC |
| 9 | 5art27 | GGAACAAGGAAUACACAGGAGACGACGGAACGAAGGACACAGACGAUAACA |
| | | AGAGCCACC |
| 10 | 5nat1 | GGAGGAACAGCAAAUUCAGGAAAAAGUGGCGACCUUUCGACUCGCCACC |
| 11 | 3'UTR | CUCGAGCUGGUACUGCAUGCACGCAAUGCUAGCUGCCCCUUUCCCGUCCUGG GUACCCCGAGUCUCCCCCGACCUCGGGUCCCAGGUAUGCUCCCACCUCCACC UGCCCCACUCACCACCUCUGCUAGUUCCAGACACCUCCCAAGCACGCAGCAA UGCAGCUCAAAACGCUUAGCCUAGCCACACCCCCACGGGAAACAGCAGUGAU UAACCUUUAGCAAUAAACGAAAGUUUAACUAAGCUAUACUAACCCCAGGGU UGGUCAAUUUCGUGCCAGCCACACCCUGGAGCUAGC |
| 12 | PolyA | AAAAAAAAAAAAAAAAAAAAAAAAAAAAAGCATATGACTAAAAAAAAAAAA AAAAAAAAAAAAAAAAAAAAAAAAAAAAAAAAAAAAAAAAAAAAAAAA AAAAAAAAA |
| 13 | HDV | GGGTCGGCATGGCATCTCCACCTCCTCGCGGTCCGACCTGGGCATCCGAAGGA GGACGCACGTCCACTCGGATGGCTAAGGGAGGGCG |
| 14 | GFP | ATGGTGAGCAAGGGCGAGGAGCTGTTCACCGGGGTGGTGCCCATCCTGGTCGA GCTGGACGGCGACGTAAACGGCCACAAGTTCAGCGTGTCCGGCGAGGGCGAG GGCGATGCCACCTACGGCAAGCTGACCCTGAAGTTCATCTGCACCACCGGCAA GCTGCCCGTGCCCTGGCCCACCCTCGTGACCACCCTGACCTACGGCGTGCAGT GCTTCAGCCGCTACCCCGACCACATGAAGCAGCACGACTTCTTCAAGTCCGCC ATGCCCGAAGGCTACGTCCAGGAGCGCACCATCTTCTTCAAGGACGACGGCAA CTACAAGACCCGCGCCGAGGTGAAGTTCGAGGGCGACACCCTGGTGAACCGC ATCGAGCTGAAGGGCATCGACTTCAAGGAGGACGGCAACATCCTGGGGCACA AGCTGGAGTACAACTACAACAGCCACAACGTCTATATCATGGCCGACAAGCAG AAGAACGGCATCAAGGTGAACTTCAAGATCCGCCACAACATCGAGGACGGCA GCGTGCAGCTCGCCGACCACTACCAGCAGAACACCCCCATCGGCGACGGCCCC GTGCTGCTGCCCGACAACCACTACCTGAGCACCCAGTCCGCCCTGAGCAAAGA CCCCAACGAGAAGCGCGATCACATGGTCCTGCTGGAGTTCGTGACCGCCGCCG GGATCACTCTCGGCATGGACGAGCTGTACAAGTGA |

### Example 3. In Vitro Preparation of Screened 5'UTR-GFP mRNAs

The 10 candidates 5'UTR-GFP plasmids preliminarily screened in Example 2 were subjected to *in vitro* transcription to prepare mRNAs.

Meanwhile, sequence V1-UTR (5con1, SEQ ID NO: 15) from patent US20200208145A1, sequence CYBA (5con2, SEQ ID NO: 16) from patent CN107849574B, globin-a (5con3, SEQ ID NO: 17) and NASAR (5con4, SEQ ID NO: 18) from the article by Zeng, C., et al., Adv. Mater. 2020, 2004452, and the 5'UTR sequence derived from the BioNTech COVID-19 vaccine BNT162b2 (Opt001, SEQ ID NO: 19) were selected as controls, and 5 control 5'UTR-GFP plasmids were constructed using the same method as in Example 2. The control 5'UTR sequences are shown in Table 2.

**Table 2. Control 5'UTR sequences**

| SEQ ID NO. | Name | Sequence |
|---|---|---|
| 15 | 5con1 | GGGAAAUAAGAGAGAAAAGAAGAGUAAGAAGAAAUAUAAGACCCCGGCGCC GCCACC |
| 16 | Scon2 | GGGCGCGCCUAGCAGUGUCCCAGCCGGGUUCGUGUCGCCGCCACC |
| 17 | Scon3 | GGGACUCUUCUGGUCCCCACAGACUCAGAGAGAACGCCACC |
| 18 | Scon4 | GGCAAAAAUCAAAAUCAAUCAUCAUCACAACAUCAACAAUCAAUCAUCAAC ACAUCAUCAAGACACCACC |
| 19 | Opt001 | GAGAAUAAACUAGUAUUCUUCUGGUCCCCACAGACUCAGAGAGAACCCGCC ACC |

The 15 plasmids described above were linearized and purified to obtain *in vitro* transcription templates, and subsequently, 15 mRNA molecules were prepared through *in vitro* transcription (IVT), capping, and purification.

The specific steps were as follows:
(1) Plasmid linearization and enzyme digestion were performed according to the following enzyme digestion volumes. 37 °C, 2 h.

**Table 3. Plasmid enzyme digestion system**

| Component | Volume |
|---|---|
| Plasmid (1mg/ml) | 50µl (50µg) |
| NotI(20U/µl)(NEB) | 6µl (2.4U/µg) |
| 10×Digestion buffer(NEB) | 20 µl |
| RNase-free H2O | To 200 µl |

(2)Purification via ion-exchange chromatography medium: 2.5 µL of ion-exchange chromatography medium (Suzhou Ruina, 04051-010000) and 600 µL of DNA-binding buffer were added, the mixture was incubated slowly on a mixer for 20-30 min and centrifuged at 3000 g for 3 min, and the supernatant was discarded; an appropriate amount of 70% ethanol was used to slowly resuspend into a suspension, which was centrifuged at 3000 g for 3 min, and the supernatant was discarded; the tube was inverted on paper for 1-2 min and placed in a clean bench for 3-5 min until the ethanol evaporated, and 30 µL of water was added.
(3) *In vitro* transcription (IVT): incubation at 32 °C for 2 h; addition of 10 µL of 1 U/µL DNase I: a final concentration of 0.1 U/µL, digestion at 37 °C for 15 min.

**Table 4. IVT reaction system**

| Reaction component | Volume |
|---|---|
| 1 M Tris-HCl | 4 µl (40 mM) |
| 1M MgCl₂ | 2.5 µl (25 mM) |
| 1 M DTT solution | 1µl(10 mM) |
| 20mM spermidine | 10µl(2 mM) |
| 100 mM ATP solution | 7 µl(7 mM) |
| 100 mM CTP solution | 7 µl(7 mM) |
| 100 mM GTP solution | 7 µl(7 mM) |
| 100 mM m1ψ solution | 7 µl(7 mM) |
| DNA template | 5µg |
| 40U/µl Ribonuclease Inhibitor | 5µl(2U/µl) |
| 0.1U/ml Inorganic Pyrophosphatase | 8 µl(0.0008U/ml) |
| 50U/µl T7 RNA polymerase | 16µl(8U/µl) |
| RNase-free water | To 100µl |

(4) Purification of IVT mRNA via ion-exchange chromatography medium: 2.5 µL of ion-exchange chromatography medium (Suzhou Ruina, 04051-010000) and 200 µL of RNA-binding buffer were added, the mixture was incubated slowly on a mixer for 20-30 min and centrifuged at 3000 g for 3 min, and the supernatant was discarded; an appropriate amount of 70% ethanol was used to slowly resuspend into a suspension, which was centrifuged at 3000 g for 3 min, and the supernatant was discarded; the tube was inverted on paper for 1-2 min and placed in a clean bench for 3-5 min until the ethanol evaporated, and an appropriate amount of water was added.
(5) mRNA capping: 37 °C, 1 h.

**Table 5. Capping system**

| Reaction component | |
|---|---|
| 1× capping buffer | 100 µl |
| 100 mM GTP solution | 5 µl(0.5 mM) |
| IVT mRNA(0.5mg/ml) | 0.5mg |
| 40U/µl Ribonuclease Inhibitor | 12.5µl(0.5U/µl) |
| 10U/µl Vaccinia capping Enzyme | 50µl(0.5U/µl) |
| 50U/µl 2-O-Methyltransferase | 50µl(2.5U/µl) |
| 32mM SAM | 6.25 µl (200 mM) |
| Reaction component | |
| RNase-free water | To 1ml |

(6) Purification via ion-exchange chromatography medium: 3 µL of ion-exchange chromatography medium (Suzhou Ruina, 04051-010000) and 2 mL of RNA-binding buffer were added, the mixture was incubated slowly on a mixer for 20-30 min and centrifuged at 3000 g for 3 min, and the supernatant was discarded; an appropriate amount of 70% ethanol was used to slowly resuspend into a suspension, which was centrifuged at 3000 g for 3 min, and the supernatant was discarded; the tube was inverted on paper for 1-2 min and placed in a clean bench for 3-5 min until the ethanol evaporated, and 300 µL of water was added. After the RNA concentration was measured, RNA integrity was determined by capillary electrophoresis (CE).

### Example 4. Comparison of Protein Expression Levels of Different 5'UTR-mRNA Molecules in 293T Cells

The 15 mRNA molecules prepared in Example 3 were transfected into 293T cells to compare their protein expression levels in 293T cells. The specific experimental method was as follows.

About 18-24 h before transfection, 0.5 mL of complete growth medium was placed into each well of a 24-well culture dish. The cell density before transfection was ≥80%. The TransIT^{®}-mRNA Transfection Kit (Mirus, MIR2256) is a transfection kit that can transfect mRNA into various cells for protein translation, and it contains two components, namely: RNA Boost Reagent and TransIT-mRNA Reagent, which were equilibrated to room temperature and gently vortexed before use. 50 µL of Opti-MEM I medium was pipetted into a sterile tube. 0.5 µg (e.g., 0.5 µL pipetted from a 1 µg/µL stock solution) of the mRNA prepared in Example 3 was added. The mixture was gently pipetted until completely mixed. 1 µL of mRNA Boost Reagent was added to the diluted RNA mixture. The mixture was gently pipetted until completely mixed. 1 µL of TransIT-mRNA Reagent was added to the diluted RNA mixture, and the mixture was gently pipetted until completely mixed. The mixture was incubated at room temperature for 2-5 min to allow sufficient time for complex formation, then the complex was gently dropped into the cell culture well, and the cell plate was gently shaken to mix well. Microscopic observation (parameters: ocular lens: 10×, objective lens: 10×, exposure time: 600 ms) and flow cytometry detection (parameters: FSC: 60; SSC: 300; BL1: 265) were performed at 24 h after transfection.

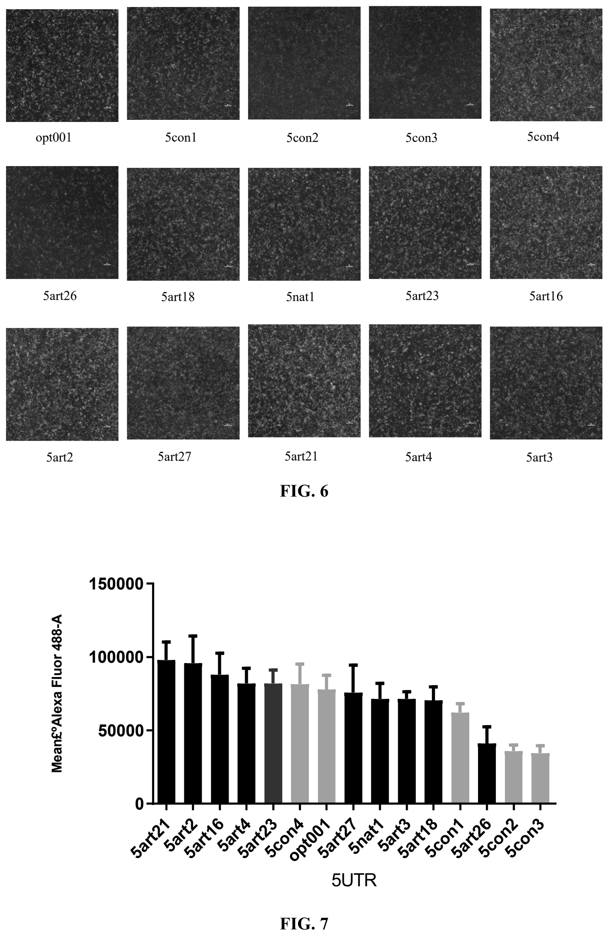

Green fluorescent protein expression could be observed for all 5'UTR-mRNA molecules after 24 h by a fluorescence microscope, as shown in FIG. 6. A positive cell population was detected by a flow cytometer, and the mean fluorescence intensity of the positive cell population from three replicate experiments was calculated. The results are shown in FIG. 7. The mean fluorescence intensity of 5art21, 5art2, 5art16, 5art4, and 5art23 were all higher than that of all control 5'UTR sequences and were more than 2 times higher than that of the control sequences 5con2 and 5con3, showing a higher protein expression level.

### Example 5. Comparison of Protein Expression Levels of Different 5'UTR-mRNA Molecules in BHK21 Cells

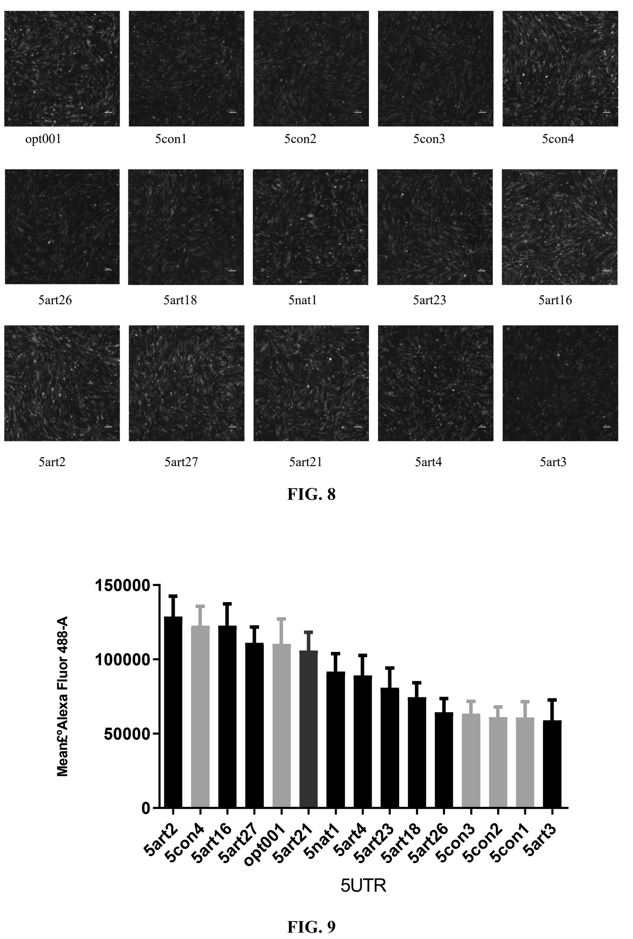

The 15 mRNA molecules prepared in Example 3 were transfected into BHK21 cells to compare their protein expression levels in BHK21 cells. The specific experimental method was as follows. BHK21 cells were evenly distributed into a 24-well plate at 1-2 × 10⁵ cells/well, and the cell density was ≥80% after overnight culture. The transfection was performed using the TransIT^{®}-mRNA Transfection Kit, following the same method as in Example 4. Microscopic observation (parameters: ocular lens: 10×, objective lens: 10×, exposure time: 600 ms) and detection with a flow cytometer (parameters: FSC: 60; SSC: 310; BL1: 285) were performed at 24 h after transfection. The results are shown in FIG. 8. Green fluorescent protein expression could be observed for all 5'UTR-mRNA molecules after 24 h by a fluorescence microscope. A positive cell population was detected by a flow cytometer, and the mean fluorescence intensity of the positive cell population from three replicate experiments was calculated. The results are shown in FIG. 9. 5art2 showed the highest protein expression level, which was higher than that of all control sequences. The mean fluorescence intensities of 5art2 and 5art16 were both more than 2 times higher than that of the control 5con2, and the mean fluorescence intensities of 5art27, 5art21, and 5nat1 were all more than 1.5 times higher than that of the control 5con2.

### Example 6. Comparison of Protein Expression of Different 5'UTR-mRNA Molecules in 293T Cells at Different Time Points

mRNA molecules prepared in Example 3 were transfected into 293T cells to compare their protein expression levels in 293T cells at different time points. The specific experimental method was as follows.

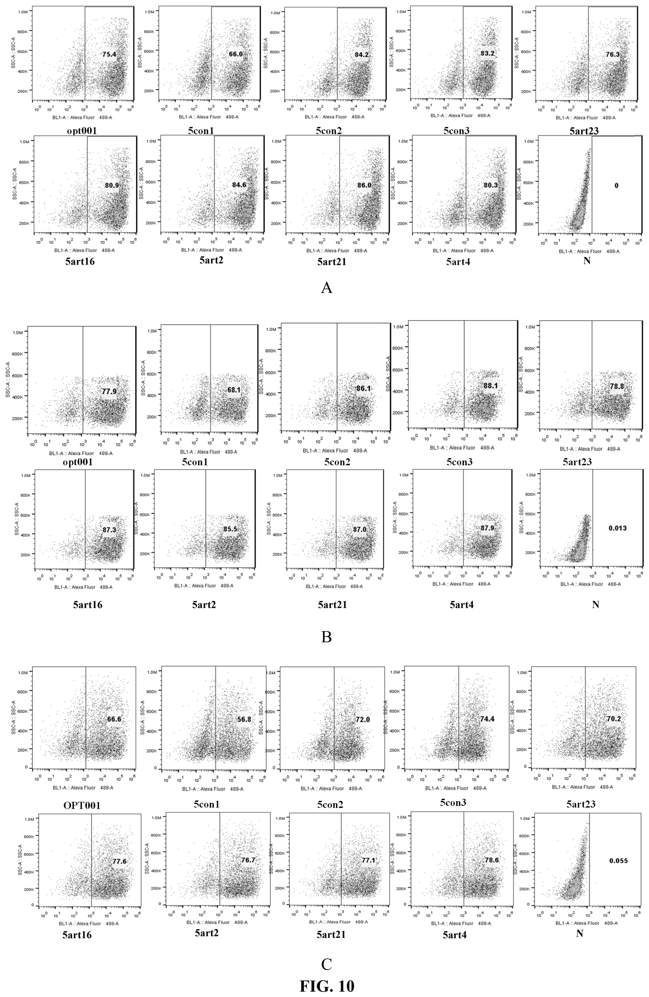

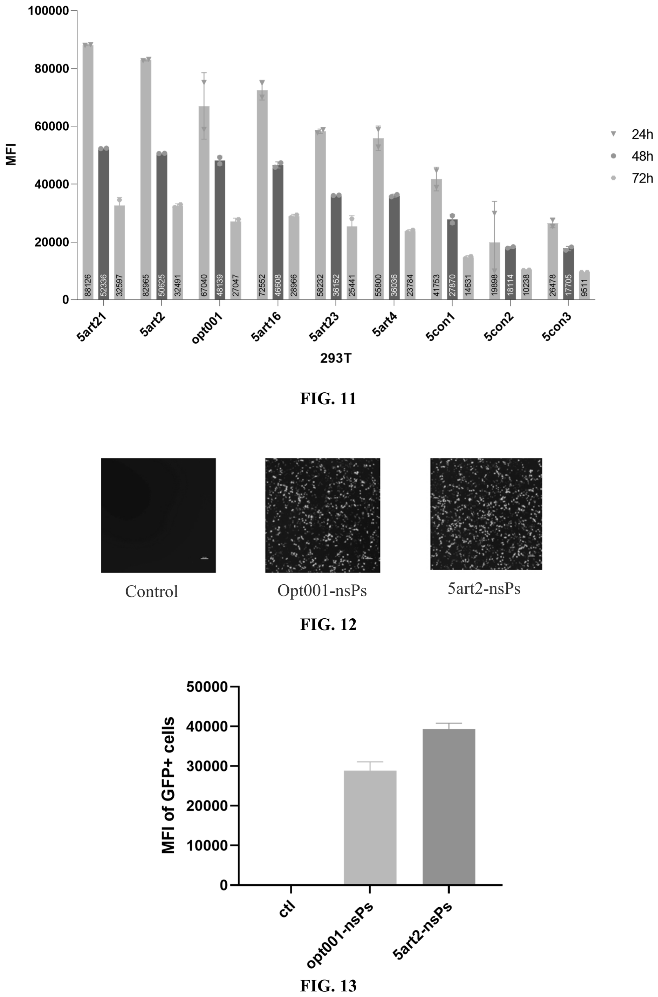

293T cells were evenly distributed into a 24-well plate and cultured overnight, and when the cell density reached ≥80%, transfection was performed using the transIT^{®}-mRNA Transfection Kit, following the same method as in Example 4. Microscopic observation and detection with a flow cytometer were performed at 24 h, 48 h, and 72 h after transfection, with parameters the same as in Example 4. The results are shown in FIGs. 10A, 10B, and 10C. 293T cells transfected with different 5'UTR-mRNA molecules could express the target protein at different time points. FIG. 11 shows that at different time points, the mean fluorescence intensities of 5art21 and 5art2 were both higher than that of the control opt001. At different time points, the mean fluorescence intensities of 5art21, 5art2, and 5art16 were all more than 2 times higher than that of the control 5con2. N represents 293T cells not transfected with mRNA.

### Example 7. Comparison of Protein Expression of Different Trans-Self-Replicating 5'UTR-nsPs Molecules in 293T Cells

Trans-amplifying mRNA (trans-amplifying RNA, taRNA) is a novel mRNA vaccine structure generated in the self-replication process of self-amplifying mRNA. This system uses two mRNAs: one mRNA encodes the gene sequence of the target protein, while the other mRNA encodes the replicase system required for self-replication, and the system consists of 4 nonstructural proteins (NSPs). These 4 nonstructural proteins (NSP1-4) can form a complex with RNA-dependent RNA polymerase activity, amplifying the mRNA encoding the target protein in the cytoplasm in a trans manner. 5'UTR-nsPs and taRNA-GFP were constructed by using the trans-amplifying system to express the replicase system and the target protein GFP, respectively. The two mRNAs were co-transfected into cells to compare the difference in target protein expression between candidate 5'UTRs and control 5'UTRs in 293T cells. The specific method was as follows.

The sequences of the T7 promoter, 5'UTR (5art2 or opt001), NSPs (NSP1-4, derived from the Venezuelan equine encephalitis virus TC-83 strain, NCBI accession number MZ399799.1), 3'UTR (see SEQ ID NO: 11), and polyA (SEQ ID NO: 12) were synthesized in order by gene synthesis and inserted into the pUC57 plasmid to construct pUC57-5art2-nsPs and pUC57-opt001-nsPs plasmids. Simultaneously, the sequences of the T7 promoter, 5'vUTR, 5'CSE and SGP, GFP (SEQ ID NO: 14), 3'CSE, and PolyA (5'vUTR, 5'CSE, SGP, and 3'CSE are all derived from the Venezuelan equine encephalitis virus TC-83 strain; PolyA consists of 75 A) were synthesized in order and inserted into the pUC57 plasmid to construct pUC57-taRNA-GFP. The 3 plasmids described above were then transcribed *in vitro* into Opt001-nsPs, 5art2-nsPs, and taRNA-GFP mRNAs for later use.

293T cells were evenly distributed into a 12-well plate and cultured overnight, and when the cell density reached ≥80%, transfection was performed using the transIT^{®}-mRNA Transfection Kit. Either 0.5 µg of 5art2-nsPs or 0.5 µg of opt001-nsPs RNA was co-transfected with 0.5 µg of taRNA-GFP into the 12-well plate, following the same method as in Example 4. Microscopic observation and detection with a flow cytometer were performed at 24 h after transfection. The results are shown in FIG. 12. Stronger fluorescence for 5art2 compared with opt001 was observed after 24 h by a fluorescence microscope. A positive cell population was detected by a flow cytometer, and the mean fluorescence intensity of the positive cell population was calculated. The results are shown in FIG. 13. Compared with opt001, 5art2 showed a stronger translational capacity. Control represents 293T cells not transfected with mRNA.

### Example 8. Construction and Preparation of 5'UTR-MICA/B mRNA Molecules, and Comparison of Their Protein Expression in BHK21 Cells

### 8.1. Construction and preparation of 5'UTR-MICA/B mRNA molecules

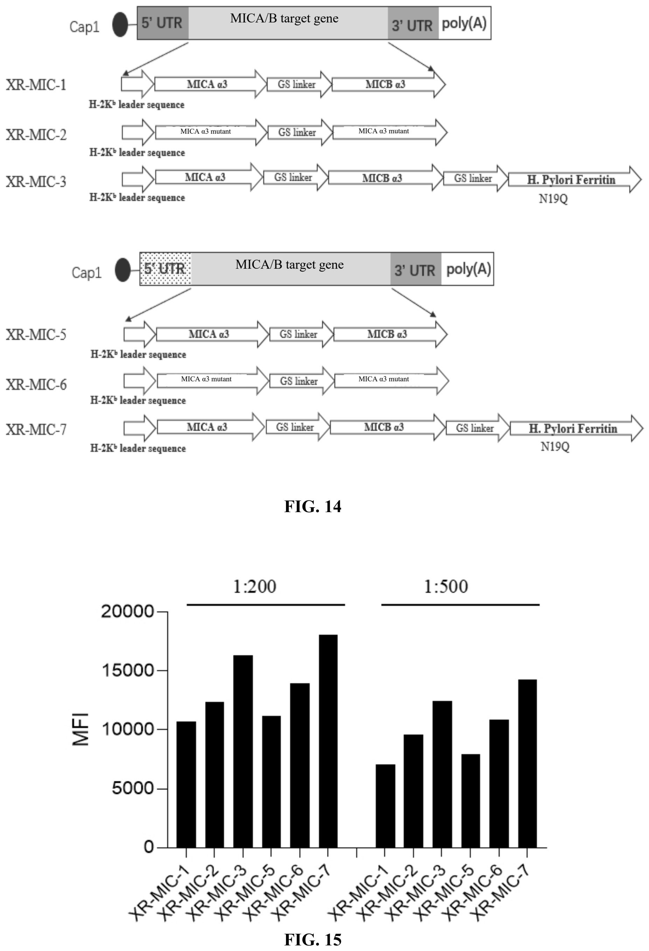

MICA/B is highly expressed on the surface of various tumor cells, such as non-small cell lung cancer, colon cancer, breast cancer, etc., and is a tumor-associated antigen. As shown in FIG. 14, 6 constructs, XR-MIC-1 to 7, targeting the MICA/B protein were constructed. Based on the difference of the 5'UTRs, the constructs were divided into two groups: the control group using Opt001 (XR-MIC-1 to XR-MIC-3) and the candidate group using 5art2 (XR-MIC-5 to XR-MIC-7). The target gene sequences for MICA/B are set forth in SEQ ID NOs: 20-22, where SEQ ID NO: 20 is adopted for XR-MIC-1 and XR-MIC-5, SEQ ID NO: 21 is adopted for XR-MIC-2 and XR-MIC-6, and SEQ ID NO: 22 is adopted for XR-MIC-3 and XR-MIC-7. The 3'UTR sequence is set forth in SEQ ID NO: 11, and the polyA sequence is set forth in SEQ ID NO: 12. The MICA/B target gene sequences for the 6 constructs are shown in Table 6.

**Table 6. Sequences of MICA/B target gene in constructs XR-MIC-1 to 7**

| SEQ ID NO. | Name | Nucleic acid sequence of MICA/B target gene |
|---|---|---|
| 20 | XR-MIC-1/ XR-MIC-5 | atggtcccctgcacactgctgctgctgctggccgccgcactggctccaactcagactcgggcacgaaccgtccctccaa tggtcaacgtgacccggtctgaggccagcgagggcaacatcaccgtgacatgccgggccagctccttctaccctagaa atatcatcctgacatggaggcaggacggcgtgtccctgtctcacgatacccagcagtggggcgacgtgctgccagatg gcaacggcacctaccagacatgggtggcaaccaggatctgcaggggcgaggagcagaggttcacatgttatatggag cacagcggcaatcactccacccacccagtgccctctggcaaggtgctggtgctgcagagccactggcagacatttcac ggcggcggcggctctggaggaggaggaagcggaggaggaggctcccgcaccgtgccccctatggtgaacgtgaca tgctccgaggtgtctgagggcaatatcaccgtgacatgtcgcgcctctagcttctatccccggaacatcaccctgacatg gagacaggacggcgtgagcctgtcccacaacactcagcagtggggagacgtgctgcctgatggcaatggaacttacc agacatgggtggccacacggatcagacagggcgaggagcagagatttacctgttatatggagcactccggcaatcacg gcacccatcccgtcccctctggcaaagccctggtcctgcagtctcagcgaaccgatttcccctgataatag |
| 21 | XR-MIC-2/ XR-MIC-6 | atggtcccttgcacactgctgctgctgctggctgctgccctggctccaactcagactcgcgcccgaaccgtccctccaat ggtccaggtgacacggtctgaggccagcgagggccagatcaccgtgacatgccgggccagctccttctaccctagaa acatcatcctgacatggaggcaggacggcgtgtccctgtctcacgatacccagcagtggggagacgtgctgccagatg gacagggcacctaccagacatgggtggcaaccaggatctgcaggggcgaggagcagaggttcacatgttatatggag cacagcggacagcactccacccacccagtgccctctggcaaggtgctggtgctgcagagccactggcagacctttcac ggaggaggaggatctggaggaggaggaagcggcggaggaggctcccgcaccgtgccccctatggtgcaggtgaca tgctccgaggtgtctgaaggacagatcaccgtgacatgtcgcgcctctagcttctatccccggaacatcaatctgacatg gagacaggacggcgtgagcctgtcccacaacactcagcagtggggcgatgtgctgcctgacggccagggaacttacc agacatgggtggccacacggatcagacagggcgaggagcagagatttacctgttatatggagcactccggccagcac ggcacccaccccgtcccaagcggcaaagccctggtcctgcagtcacagcgaacagatttcccttgataatag |
| 22 | XR-MIC-3/ XR-MIC-7 | atggtcccttgtaccctgctgctgctgctggctgctgccctggccccaactcagactcgcgctcgcactgtcccacctatg gtcaacgtgacccggtccgaggcctctgagggcaacatcaccgtgacatgccgggccagctccttctacccaagaaat atcatcctgacctggaggcaggacggcgtgtctctgagccacgatacacagcagtggggagacgtgctgccagatgg aaacggcacctaccagacatgggtggcaaccaggatctgcaggggcgaggagcagcggttcacatgttatatggagc actctggcaatcacagcacccacccagtgccatccggaaaggtgctggtgctgcagtctcactggcagacctttcacgg aggaggaggatccggaggaggaggatctggcggcggcggcagcagaaccgtgccccctatggtgaacgtgacatg cagcgaggtgtccgagggcaatatcaccgtgacatgtcgcgcctctagcttttatcctcggaacatcaccctgacatgga |
| | | gacaggacggcgtgtccctgtctcacaacacccagcagtggggcgacgtgctgccagatggcaatggaacttaccag acatgggtggccacacggatcagacagggcgaggagcagaggttcacctgctatatggagcacagcggcaatcacg gcacacaccctgtgccaagcggaaaggccctggtgctgcagtcccagaggaccgactttccaggcggcggcggcag cggcggcggcggctccggcggcggcggcagcgatatcatcaagctgctgaacgagcaagtgaataaggagatgca gtcctctaacctgtacatgtctatgagctcctggtgttatacacactccctggacggagcaggcctgttcctgtttgatcacg ccgccgaggagtacgagcacgccaagaagctgatcatcttcctgaatgagaacaatgtgcccgtgcagctgatcagca tctccgcccctgagcacaagttcgagggcctgacccagatctttcagaaggcctacgagcacgagcagcacatctctga gagcatcaacaatatcgtggaccacgccatcaagtccaaggatcacgccacattcaactttctgcagtggtacgtggccg agcagcacgaggaggaggtgctgtttaaggacatcctggataagatcgagctgatcggcaatgagaaccacggcctgt acctggcagaccagtatgtcaagggaatcgcaaagtcacgcaaaagctgataatag |

The corresponding mRNA molecules for the constructs described above were obtained through *in vitro* transcription, following the same method as in Example 3. The concentration, yield, and integrity of the obtained mRNA molecule stock solutions were determined using conventional methods in the art, as shown in Table 7. It can be seen that the integrity of all constructs exceeded 90%, and the concentration and yield of mRNAs using 5art2 were superior to those of mRNAs using Opt001.

**Table 7. Detection of stock solutions of XR-MIC construct mRNA molecules**

| | **XR-MIC-1** | **XR-MIC-2** | **XR-MIC-3** | **XR-MIC-5** | **XR-MIC-6** | **XR-MIC-7** |
|---|---|---|---|---|---|---|
| **Concentration (mg/mL)** | 0.812 | 0.9 | 1.468 | 1.89 | 1.723 | 2.375 |
| **mRNA yield (mg/mL)** | 2.9 | 3.21 | 4.58 | 4.32 | 4.93 | 5.09 |
| **CE integrity (%)** | 96.3 | 95.4 | 94.6 | 94.6 | 95 | 94.7 |

### 8.2. Comparison of protein expression of different 5'UTR-MICA/B mRNA molecules in BHK21 cells

The mRNA molecules of XR-MIC-1 to XR-MIC-7 obtained above were transfected into BHK21 cells, following the same transfection method as in Example 4. Cells were harvested 48 h later for fixation and permeabilization, with steps as follows:
1. Preparation of fixation/permeabilization solution: 1 mL of Fixation/Permeabilization Concentrate (Thermo fisher, 00-5123) was added to 3 mL of Fixation/Permeabilization Diluent (Thermo fisher, 00-5223). 48 h after transfection, 200 µL of the prepared fixation/permeabilization solution was added, 40 min.
2. Preparation of permeabilization buffer (PB): 1 mL of 10× Permeabilization Buffer (Thermo fisher, 00-8333) was added to 9 mL of ddH2O.
3. The supernatant was discarded after centrifugation, 200 µL of permeabilization buffer (PB) was added, and after 5 min, fetal bovine serum (final concentration: 2%) was added for blocking at room temperature for 15 min.
4. 200 µL of MICA mouse monoclonal 6G6 antibody (Santa Cruz, sc-56995) diluted with permeabilization buffer at a ratio of 1:200 or 1:500 was added, and the mixture was incubated for 1 h.
5. The supernatant was discarded after centrifugation, 200 µL of permeabilization buffer (PB) was added, and the plate was washed twice.
6. Goat anti-mouse FITC secondary antibody (Thermo fisher, A16079) diluted with permeabilization buffer at a ratio of 1:800 was added, and the plate was incubated for 30 min.
7. The supernatant was discarded after centrifugation, and 200 µL of permeabilization buffer (PB) was added; the mixture was washed twice, and 200 µL of PBS was added for detection with an Attune NxT acoustic focusing flow cytometer (Thermo, 2AFC236901121).

The results are shown in FIG. 15. Whether the MICA mouse monoclonal 6G6 antibody was diluted at a ratio of 1:200 or 1:500, the mean fluorescence value of XR-MIC-5 was higher than that of XR-MIC-1, the mean fluorescence value of XR-MIC-6 was higher than that of XR-MIC-2, and the mean fluorescence value of XR-MIC-7 was higher than that of XR-MIC-3. It can be seen that the expression efficiency of 5art2 is superior to that of Opt001.

## Claims

1. An engineered mRNA, comprising:
a first nucleic acid sequence, wherein the first nucleic acid sequence comprises at least one engineered 5' untranslated region (5'UTR) sequence;
a second nucleic acid sequence, wherein the second nucleic acid sequence comprises at least one open reading frame (ORF); and
a third nucleic acid sequence, wherein the third nucleic acid sequence comprises at least one 3' untranslated region (3'UTR) sequence.

2. The engineered mRNA according to claim 1, wherein the engineered 5'UTR sequence comprises a sequence represented by formula (I) or formula (II) below: 5'-GG(N)ₙGCCACC-3' (I) (SEQ ID NO:23); 5'-GG(H)ₙGCCACC-3' (II) (SEQ ID NO:24), wherein
each N is independently selected from A, U, G, or C;
each H is independently selected from A, U, or C;
n represents the number of N or H and is any integer of 30-70.

3. The engineered mRNA according to claim 2, wherein formula (I) or formula (II) has a length of 60 nt or 70 nt, a GC content of 50-60%, and a minimum free energy of 0.

4. The engineered mRNA according to any one of claims 1-3, wherein the engineered 5'UTR sequence is selected from the group comprising the following: SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, or a sequence having at least 90% identity to the sequences set forth in SEQ ID NOs: 1-10 or having 1, 2, 3, or more nucleotide insertions, deletions, and/or substitutions.

5. The engineered mRNA according to any one of claims 1-4, wherein the 5'UTR sequence is suitable for increasing protein production from the mRNA.

6. The engineered mRNA according to any one of claims 1-5, wherein the 3'UTR sequence is selected from SEQ ID NO: 11.

7. The engineered mRNA according to any one of claims 1-6, wherein the at least one 3'UTR sequence and the at least one 5'UTR sequence act synergistically to increase protein production from the mRNA.

8. The engineered mRNA according to any one of claims 1-7, wherein the 5'UTR sequence, the open reading frame, and the 3'UTR sequence are heterologous.

9. The engineered mRNA according to any one of claims 1-8, wherein the open reading frame encodes a target protein, the target protein is a protein with biological activity or a protein with chromogenic/indicator functions, and the source of the target protein includes, but is not limited to, viruses, bacteria, and eukaryotes.

10. The engineered mRNA according to any one of claims 1-9, wherein the mRNA further comprises a 5' cap structure and a polyA sequence.

11. A vector, comprising a nucleic acid encoding the engineered mRNA according to any one of claims 1-10.

12. The vector according to claim 11, wherein the vector is an RNA vector or a DNA vector.

13. The vector according to claim 11, wherein the vector is a plasmid vector or a viral vector.

14. The vector according to any one of claims 11-13, wherein the vector can further comprise an expression control element.

15. A cell, comprising the vector according to any one of claims 11-14, wherein preferably, the cell is a prokaryotic cell or a eukaryotic cell, including but not limited to a bacterium (*E. coli*), a fungus (yeast), an insect cell, or a mammalian cell.

16. A composition, comprising the engineered mRNA according to any one of claims 1-10 and an mRNA delivery system, wherein preferably, the delivery system comprises a lipid nanoparticle, a complex and a polymer nanoparticle, an exosome, a biomicrovesicle, protamine, and the like.

17. A pharmaceutical composition, comprising the engineered mRNA according to any one of claims 1-10, the vector according to any one of claims 11-14, the cell according to claim 15, or the composition according to claim 16, and a pharmaceutically acceptable carrier.

18. Use of the engineered mRNA according to any one of claims 1-10, the vector according to any one of claims 11-14, the cell according to claim 15, the composition according to claim 16, or the pharmaceutical composition according to claim 17 in preparing a medicament for treating or preventing a disease by administering to a subject in need thereof.

19. A method for treating or preventing a disorder, comprising administering to a subject in need thereof the engineered mRNA according to any one of claims 1-10, the vector according to any one of claims 11-14, the cell according to claim 15, the composition according to claim 16, or the pharmaceutical composition according to claim 17.

20. A method for treating or preventing a disorder, comprising transfecting a cell with the engineered mRNA according to any one of claims 1-10 or the vector according to any one of claims 11-14.

21. The engineered mRNA according to any one of claims 1-10, the vector according to any one of claims 11-14, the cell according to claim 15, the composition according to claim 16, or the pharmaceutical composition according to claim 17 for use as a medicament, a vaccine, or a gene therapy.

22. A method for increasing protein expression, comprising providing the engineered mRNA according to any one of claims 1-10, the vector according to any one of claims 11-14, or the cell according to claim 15 to a cell or tissue.
